# EUROPEAN PATENT APPLICATION

(11) **EP 4 360 576 A1**
(43) Date of publication of application: **01.05.2024**
(21) Application number: 23200662.7
(22) Date of filing: 28.09.2023
(51) Int. Cl.: A61B 18/14

(54) **SYSTEM OF CONTROL OF CONDUCTIVE FLUID DURING COAGULATION**

(30) Priority: 28.10.2022 US 202263381405 P
(71) Applicant: Smith & Nephew, Inc., Memphis, TN 38116 (US); Smith & Nephew Orthopaedics AG, 6300 Zug (CH); Smith & Nephew Asia Pacific Pte Limited, Queenstown 138565 (SG)
(72) Inventor: LYNN, Christopher J., Austin, 78735 (US); GOODE, Johnson E., Austin, 78733 (US); EVANS, Douglas G., Austin, 78749 (US)
(74) Representative: White, Andrew John

(57) **Abstract**

Method and system of control of conductive fluid during coagulation using radio frequency energy. At least one example is of sealing blood vessels, the method comprising: applying, by an electrosurgical controller, radio frequency (RF) energy between two electrodes of a coagulation wand, the two electrodes abutting tissue at a surgical site, and the RF energy at a coagulation energy; supplying, by the electrosurgical controller, a flow of conductive fluid to the surgical site; measuring, by the electrosurgical controller, a value indicative of impedance of tissue and conductive fluid between the two electrodes; and decreasing, by the electrosurgical controller, the flow of conductive fluid as the value indicative of impedance decreases.

## Description

### CROSS-REFERENCE TO RELATED APPLICATIONS

Not Applicable.

### BACKGROUND

Coagulation devices, and in particular those used for arthroplasty, include saline delivery to the active tip of the device and thus to the tissue at the target site. In certain situations, the saline may pool at the tip of the device. The saline is heated by the coagulation energy, and runoff of the heated saline may damage healthy tissue.

### SUMMARY

One example is a method of sealing blood vessels, the method comprising: applying, by an electrosurgical controller, radio frequency (RF) energy between two electrodes of a coagulation wand, the two electrodes abutting tissue at a surgical site, and the RF energy at a coagulation energy; supplying, by the electrosurgical controller, a flow of conductive fluid to the surgical site; measuring, by the electrosurgical controller, a value indicative of impedance of tissue and conductive fluid between the two electrodes; and decreasing, by the electrosurgical controller, the flow of conductive fluid as the value indicative of impedance decreases.

The example method may further comprise ceasing the flow of conductive fluid when the value indicative of impedance falls below a predetermined threshold. The example method may further comprise generating an alarm, by the electrosurgical controller, when the value indicative of impedance remains below the predetermined threshold for a predetermined duration, the alarm indicating a clog in an aspiration tubing associated with the surgical site.

The example method may further comprise increasing, by the electrosurgical controller, the flow of conductive fluid as the value indicative of impedance increases.

In the example method, measuring the value indicative of impedance may further comprise measuring resistance between the two electrodes; and decreasing the flow of conductive fluid may further comprise decreasing the flow of conductive fluid as the resistance decreases.

In the example method, decreasing the flow of conductive fluid may further comprise at least one selected from a group comprising: decreasing directly proportional to the value indicative of impedance; decreasing linearly proportional to the value indicative of impedance; decreasing step-wise proportional to the value indicative of impedance.

In the example method, applying the RF energy may further comprise decreasing the RF energy as the value indicative of impedance decreases.

In the example method, measuring the value indicative of impedance may further comprise measuring at least one selected from a group comprising: impedance; the real part of the impedance; resistance; electrical current flow for constant voltage surgical controller providing the RF energy.

Yet another example is an electrosurgical controller comprising: a connector accessible on an external surface of a housing, the connector defines two electrical pins; a radio frequency (RF) voltage generator disposed within the housing, and electrically coupled to the two electrical pins of the connector; a measurement circuit disposed within the housing and electrically connected between the RF voltage generator and the two electrical pins of the connector, the measurement creates a signal indicative of impedance; a pump system configured to pump conductive fluid to a surgical site; and a controller disposed within the housing and communicatively coupled to the measurement circuit and the pump system. The controller may be configured to: command the RF voltage generator to supply RF energy across the two electrical pins of the connector, and the RF energy at a coagulation energy; command the pump system to supply a flow of conductive fluid; read the signal indicative of impedance from the measurement circuit and create a value indicative of impedance; and command the pump system to decrease the flow of conductive fluid as the value indicative of impedance decreases.

In the example electrosurgical controller, the controller may be further configured to command the pump system to cease the flow of conductive fluid when the value indicative of impedance falls below a predetermined threshold.

In the example electrosurgical controller, the controller may be further configured to generate an alarm when the value indicative of impedance remains below the predetermined threshold for a predetermined duration.

In the example electrosurgical controller, when the controller reads the signal indicative of impedance and creates the value indicative of impedance, the controller may be further configured to create at least one selected from a group comprising: a value indicative of resistance; a value indicative the real part of the impedance; and a value indicative of electrical current.

In the example electrosurgical controller, when the controller commands the pump system to decrease the flow of conductive fluid, the controller may be further configured to command the decrease the flow of conductive fluid: directly proportional to the value indicative of impedance; linearly proportional to the value indicative of impedance; and step-wise proportional to the value indicative of impedance.

In the example electrosurgical controller, the controller may be further configured to command the pump system to increase the flow of conductive fluid as the value indicative of impedance increases.

In the example electrosurgical controller, when the controller commands the RF voltage generator to supply RF energy, the controller may be further configured to command the RF voltage generator to decrease the RF energy as the value indicative of impedance decreases.

Another example is an electrosurgical coagulation system comprising: a source of conductive fluid; a coagulation wand defining first and second electrodes disposed at a distal end of the coagulation wand and a delivery lumen associated with a distal end of the coagulation wand; and an electrosurgical controller. The electrosurgical controller may comprise: a radio frequency (RF) voltage generator electrically coupled to the first and second electrodes of the coagulation wand; an impedance sensitive circuit electrically connected between the RF generator and the first and second electrodes of the coagulation wand, the impedance sensitive circuit creates a signal indicative of impedance; a pump system fluidly coupled to the source of conductive fluid and the delivery lumen; and a controller electrically coupled to the impedance measuring circuit and the pump system. The controller may be configured to: command the RF voltage generator to supply RF energy between the first and second electrodes of the coagulation wand, and the RF energy at a coagulation energy; command the pump system to supply a flow of conductive fluid; read the signal indicative of impedance from the impedance sensitive circuit and create a value indicative of impedance; and command the pump system to decrease the flow of conductive fluid to the delivery lumen as the value indicative of impedance decreases.

In the example system, the controller may be further configured to command the pump system to cease the flow of conductive fluid to the delivery lumen when the value indicative of impedance falls below a predetermined threshold. The controller may be further configured to generate an alarm when the value indicative of impedance remains below the predetermined threshold for a predetermined duration, the alarm indicating a clog in an aspiration tubing associated with a surgical site.

In the example system, when the controller reads the signal indicative of impedance and creates the value indicative of impedance, the controller may be configured to create at least one selected from a group comprising: a value indicative of resistance; a value indicative the real part of the impedance; and a value indicative of electrical current.

In the example system, when the controller commands the pump system to decrease the flow of conductive fluid, the controller may be further configured to command the decrease of the flow of conductive fluid: directly proportional to the value indicative of impedance; linearly proportional to the value indicative of impedance; and step-wise proportional to the value indicative of impedance.

In the example system, the controller may be further configured to command the pump system to increase the flow of conductive fluid to the delivery lumen as the value indicative of impedance increases

In the example system, when the controller commands the RF voltage generator to supply RF energy, the controller may be s further configured to command the RF voltage generator to decrease the RF energy as the value indicative of impedance decreases.

### BRIEF DESCRIPTION OF THE DRAWINGS

For a detailed description of example embodiments, reference will now be made to the accompanying drawings in which:
Figure 1 shows an electrosurgical coagulation system in accordance with at least some embodiments;
Figure 2 shows a perspective view of a coagulation wand in accordance with at least some embodiments;
Figure 3 shows a partial cross-sectional side view of a surgical site and a distal end of the coagulation wand, in accordance with at least some embodiments;
Figure 4 shows a partial cross-sectional side view of a surgical site and a distal end of the coagulation wand, in accordance with at least some embodiments;
Figure 5 shows a partial cross-sectional side view of a surgical site and a distal end of the coagulation wand, in accordance with at least some embodiments;
Figure 6 shows a plot of a relationship of resistance and saline flow rate, in accordance with at least some embodiments;
Figure 7 shows a plot of an example relationship of resistance and saline flow rate, in accordance with at least some embodiments;
Figure 8 shows a plot of an example relationship of resistance and saline flow rate, in accordance with at least some embodiments;
Figure 9 shows a plot of saline flow rate and applied RF energy, in accordance with at least some embodiments;
Figure 10 shows an electrical block diagram of an electrosurgical controller in accordance with at least some embodiments; and
Figure 11 shows a method in accordance with at least some embodiments.

### DEFINITIONS

Various terms are used to refer to particular system components. Different companies may refer to a component by different names - this document does not intend to distinguish between components that differ in name but not function. In the following discussion and in the claims, the terms "including" and "comprising" are used in an openended fashion, and thus should be interpreted to mean "including, but not limited to...." Also, the term "couple" or "couples" is intended to mean either an indirect or direct connection. Thus, if a first device couples to a second device, that connection may be through a direct connection or through an indirect connection via other devices and connections.

"Coagulation energy" shall mean an applied energy at which coagulation takes place (e.g., sealing of blood vessels), but at which tissue ablation does not take place or does not predominantly take place.

"Tissue ablation" shall mean removal of biological tissues, such as by molecular disassociation using plasma. Causing cellular death, such as by resistive and/or inductive heating, shall not be considered "tissue ablation."

"Constant voltage surgical controller" shall mean a surgical controller that delivers radio frequency (RF) energy to one or more electrodes of a coagulation wand at a constant peak voltage when the voltage is applied (e.g., a predetermined voltage turned on and off at a radio frequency and with a fixed or a variable duty cycle).

"Controller" shall mean, alone or in combination, individual circuit components, an application specific integrated circuit (ASIC), a microcontroller with controlling software, a reduced-instruction-set computing (RISC) with controlling software, a digital signal processor (DSP), a processor with controlling software, a programmable logic device (PLD), a field programmable gate array (FPGA), or a programmable system-on-a-chip (PSOC), configured to read inputs and drive outputs responsive to the inputs.

### DETAILED DESCRIPTION

The following discussion is directed to various embodiments of the invention. Although one or more of these embodiments may be preferred, the embodiments disclosed should not be interpreted, or otherwise used, as limiting the scope of the disclosure, including the claims. In addition, one skilled in the art will understand that the following description has broad application, and the discussion of any embodiment is meant only to be exemplary of that embodiment, and not intended to intimate that the scope of the disclosure, including the claims, is limited to that embodiment.

Various examples are directed to control of conductive fluid during coagulation using radio frequency (RF) energy in surgical procedures. More particularly, various examples are directed to sealing of blood vessels using RF energy, and controlling the flow of conductive fluid (e.g., saline) to the surgical site based on a value indicative of impedance measured at the surgical site. More particularly still, during a coagulation procedure a value indicative of impedance (e.g., resistance) is measured between the electrodes of a coagulation wand. The flow of conductive fluid decreases as the value indicative of impedance decreases, and the flow of conductive fluid increases as the value indicative of impedance increases. In this way, the volume of conductive fluid at surgical site is reduced, and heating of the conductive fluid is reduced, which reduces the likelihood of runoff of hot conductive fluid reaching non-target and otherwise healthy tissue. In yet still other cases, both the flow of the conductive fluid and the amount of RF energy supplied are reduced to the same end. The specification now turns to an example system.

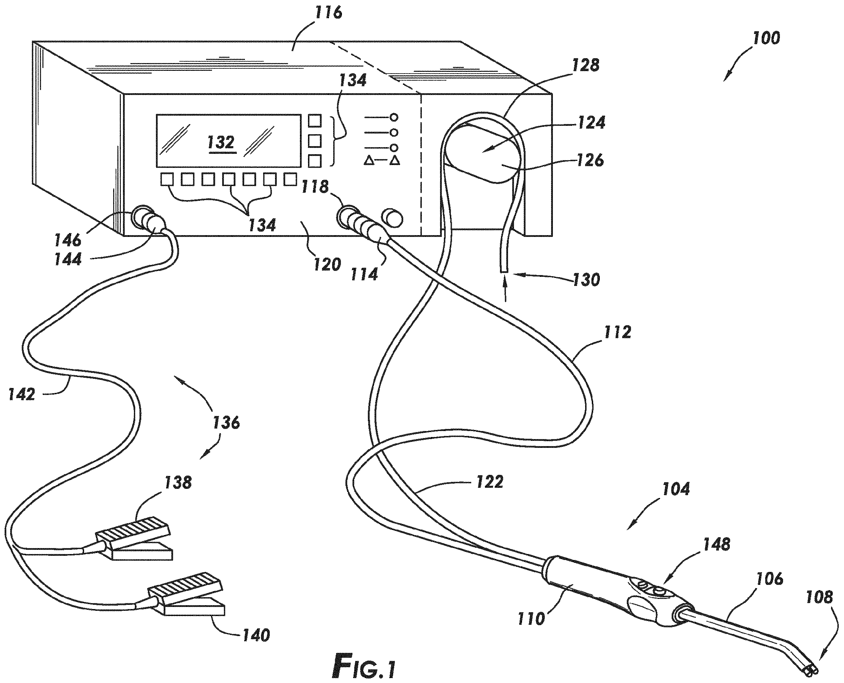

Figure 1 shows an example electrosurgical coagulation system. In particular, the electrosurgical coagulation system 100 comprises an electrosurgical coagulation device or coagulation wand 104 that comprises an elongate shaft 106 defining a distal end 108. Further, the coagulation wand 104 comprises a grip or handle 110 where a clinician holds the coagulation wand 104 during surgical procedures. The coagulation wand 104 further comprises a flexible multi-conductor cable 112 housing one or more electrical conductors or electrical leads (not specifically shown), and the flexible multi-conductor cable 112 terminates in a wand connector 114. As shown in Figure 1, the coagulation wand 104 is coupled to an electrosurgical controller 116, such as by a controller connector 118 on an outer surface of an enclosure 120 (in the illustrative case of Figure 1, the front surface of the enclosure 120).

Though not visible in the view of Figure 1, the example coagulation wand 104 has an internal flow channel or fluid delivery lumen. The fluid delivery lumen of the coagulation wand 104 is coupled to a flexible tubular member 122 used to provide conductive fluid (e.g., saline, Ringers Solution) to the distal end 108 of the coagulation wand 104. In accordance with example embodiments, the flexible tubular member 122 is coupled to a peristaltic pump 124 illustratively shown as an integral component with the electrosurgical controller 116 (i.e., residing at least partially within the enclosure 120 of the coagulation controller 116). In other embodiments, an enclosure for the peristaltic pump 124 may be separate from the enclosure 120 for the electrosurgical controller 116 (as shown by dashed lines in the figure).

The example peristaltic pump 124 comprises a rotor portion 126 (hereafter just "rotor 126") as well as a stator portion 128 (hereafter just "stator 128"). The flexible tubular member 122 is coupled within the peristaltic pump 124 between the rotor 126 and the stator 128, and movement of the rotor 126 against the flexible tubular member 122 causes fluid movement from the suction 130 toward the distal end 108 of the coagulation wand 104. While the illustrative peristaltic pump 124 is shown with a two-head rotor 126, other types of peristaltic pumps 124 may be used (e.g., a five-head peristaltic pump). In the context of the various embodiments, the peristaltic pump 124 creates a volume-controlled flow of conductive fluid to the surgical site at the distal end 108 of the coagulation wand 104 (the surgical site not specifically shown). Hereafter, "conductive fluid" will be referred as saline with the understanding that any suitable electrically conductive fluid may be used. The rate of flow of the saline is based on the rotational speed of the rotor 126, as commanded by the electrosurgical controller 116. The suction 130 may be coupled to any suitable source of saline, such as saline in a hanging bag or other container. In other cases, any pump system that provides volume controlled flow when activated (e.g., positive displacement pump, centrifugal pump with speed control) may be used.

Still referring to Figure 1, a display device or interface device 132 is visible through the enclosure 120 of the coagulation controller 116, and in some embodiments a user selects operational modes of the coagulation controller 116 by way of the interface device 132 and related buttons 134. For example, using one or more of the buttons 134 the clinician may select an initial or default flow rate for the saline. As another example, using one or more of the buttons 134 the clinician may select an applied voltage to control the aggressiveness of the coagulation.

In some embodiments the electrosurgical coagulation system 100 also comprises a foot pedal assembly 136. The foot pedal assembly 136 may comprise one or more foot pedal devices 138 and 140, a flexible multi-conductor cable 142, and a pedal connector 144. While only two foot pedal devices 138 and 140 are shown, one or more pedal devices may be implemented. The enclosure 120 of the electrosurgical controller 116 may comprise a corresponding connector 146 that couples to the pedal connector 144. A clinician may use the foot pedal assembly 136 to control various aspects of the electrosurgical controller 116. For example, foot pedal device 138 may be used for on-off control of RF energy application to the distal end 108 of the coagulation wand 104. Further, the foot pedal device 140 may be used to control and/or set the flow of saline to the distal end 108 of the coagulation wand 104. Alternatively, control of the various operational or performance aspects of the electrosurgical controller 116 (e.g., applied voltage setting) may be activated by interaction with the electrical switches or buttons 148 located on the handle 110 of the wand 102.

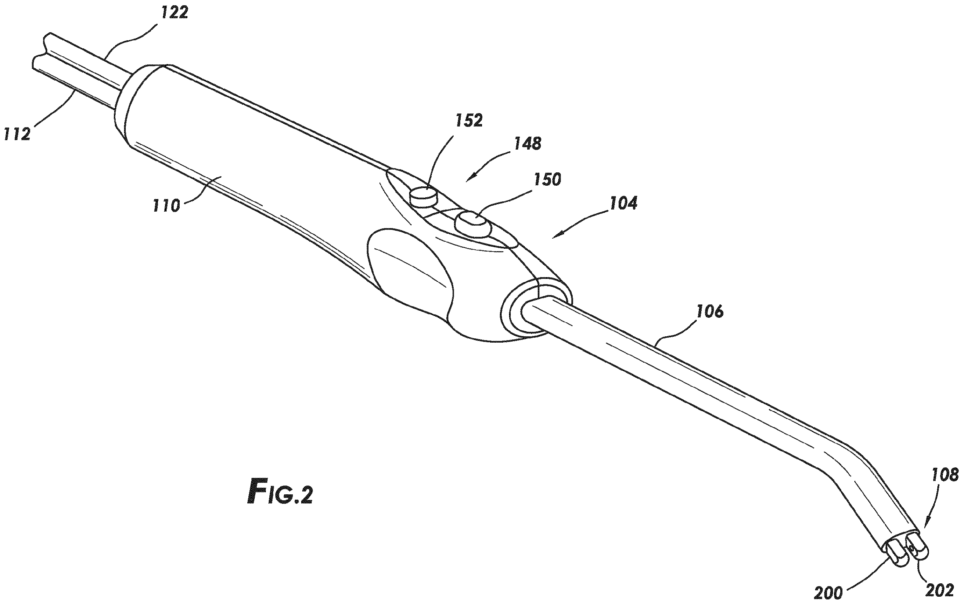

Figure 2 shows a perspective view of a coagulation wand 104 in accordance with at least some embodiments. In particular, visible in Figure 2 is the handle 110 as well as the elongate shaft 106 and distal end 108. Also visible in Figure 2 are the buttons 148 that the clinician may use to control various aspects of the operation of the coagulation wand 104 (e.g., on-off control, applied voltage setting, flow rate of saline). For example, the button 150 may control on-off state of the coagulation, while the button 152 may control an increased coagulation function. Energy is delivered to the coagulation wand 104 from the electrosurgical controller 116 (Figure 1) by way of the flexible multi-conductor cable 112, and saline is delivered by way of the flexible tubular member 122. Better visible in Figure 2 are the example electrodes at the distal end 108 of the elongate shaft 106, being an electrode 200 and an electrode 202. Each electrode is a metallic structure defining a proximal end coupled to the elongate shaft 106 and an opposite distal end that, in operation, is close to, contacts, or abuts tissue at the target site during coagulation. In example cases, each electrode 200 and 202 defines an internal irrigating fluid path or flow lumen in operational relationship to one or more apertures or nozzles. Each nozzle defines a spray direction, and the saline exits each electrode by way of and in a direction controlled by the nozzle(s). In example embodiments, the nozzles are arranged on the electrodes such that fluid exits each electrode in a direction toward the other electrode. In other cases, however, the delivery lumen through the coagulation wand 104 may have an aperture that delivers saline between or near the electrodes 200 and 202 without the saline flowing through the electrodes 200 and 202 themselves.

The RF energy applied to the electrodes 200 and 202 creates an electrical current flow between the electrodes, with the amount of electrical current based on the electrical impedance of the tissue and saline in contact with the electrodes 200 and 202. A certain amount of saline at the surgical site, partially submerging the electrodes 200 and 202, may be used to prevent charring or sticking of the electrodes to the tissue. However, saline has lower impedance than tissue, and thus not only does excess saline provide a lower impedance path for electrical current, bypassing the tissue, the electrical current that flows through the saline also heats the saline.

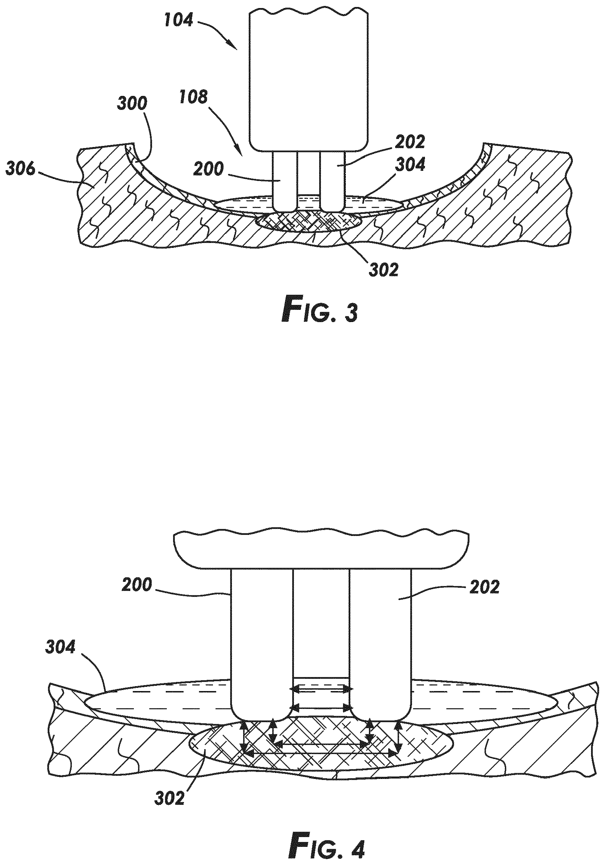

Figure 3 shows a simplified, partial cross-sectional side view, of a surgical site and a distal end of the coagulation wand. In particular, Figure 3 shows a simplified portion of the distal end 108 of the coagulation wand 104, including electrodes 200 and 202. The distal end 108 of the coagulation wand 104 is disposed within a treatment zone or surgical site 300, and the electrodes 200 and 202 abut tissue within the surgical site 300. The tissue abutted by and proximate to the electrodes 200 and 202 may be referred to as the treatment zone or coagulation zone 302 - the zone within which vessel sealing and/or coagulation predominantly takes place. The example electrosurgical controller 116 (Figure 1) delivers saline 304 to the surgical site 300, and the electrodes 200 and 202 are thus disposed at least partially within the saline 304. Outside the surgical site 300 resides skin or healthy tissue 306. Thus, the surgical site 300 of Figure 3 is an example of an open of "dry field" surgical site, such as knee or hip arthroplasty, or surgical procedures associated with the nasopharynx.

In operation, the electrosurgical controller 116 (Figure 1) applies RF energy between the electrodes 200 and 202, with an amount of energy referred to as coagulation energy. The coagulation energy is selected to be sufficient energy to heat the tissue to cause blood to coagulate and/or shrink the tissue and thus seal blood vessels. The coagulation energy may be selected from range of energies, with the selection based on the speed and aggressiveness desired by the clinician. Nevertheless, the range of energies from which the coagulation energy is selected is lower than energies to cause tissue ablation. Stated otherwise, the selected coagulation energy is an energy at which tissue ablation does not take place or does not predominantly take place. The specification now turns to a description of current flow through the tissue and saline to better describe issues that arise when excess saline is present at the surgical site 300.

Figure 4 shows a simplified, partial cross-sectional side view, of a surgical site and a distal end of the coagulation wand. In particular, Figure 4 shows example electrical current flow through coagulation zone 302 as well as the saline 304 by way of doubleheaded arrows, thereby acknowledging the alternating current (AC) nature of the RF energy. Given that the electrodes 200 and 202 abut the tissue of the coagulation zone 302 and are at least partially submerged in the saline 304, the saline 304 and tissue create parallel electrical current paths. Figure 4 illustratively shows a situation in which the amount or depth of saline (not necessarily to scale) around the electrodes 200 and 202 has a resistance to current flow about equal to the resistance to current flow through the tissue at the surgical site 300, and thus the electrical current is about equal as between the current path through the saline and the current path through the tissue at the target site. Stated otherwise, Figure 4 shows a situation in which the amount or depth of saline around the electrodes 200 and 202 has an electrical conductivity about equal to the electrical conductivity through the tissue at the surgical site 300, and thus the electrical current is about equal as between the current path through the saline and the current path through the tissue at the target site.

Saline 304 has lower resistivity to current flow than tissue, and thus the greater the depth of saline 304, the greater the electrical current flow through the saline. Stated otherwise, saline 304 has higher electrical conductivity than tissue, and thus again the greater the depth of saline 304, the greater the electrical current flow through the saline.

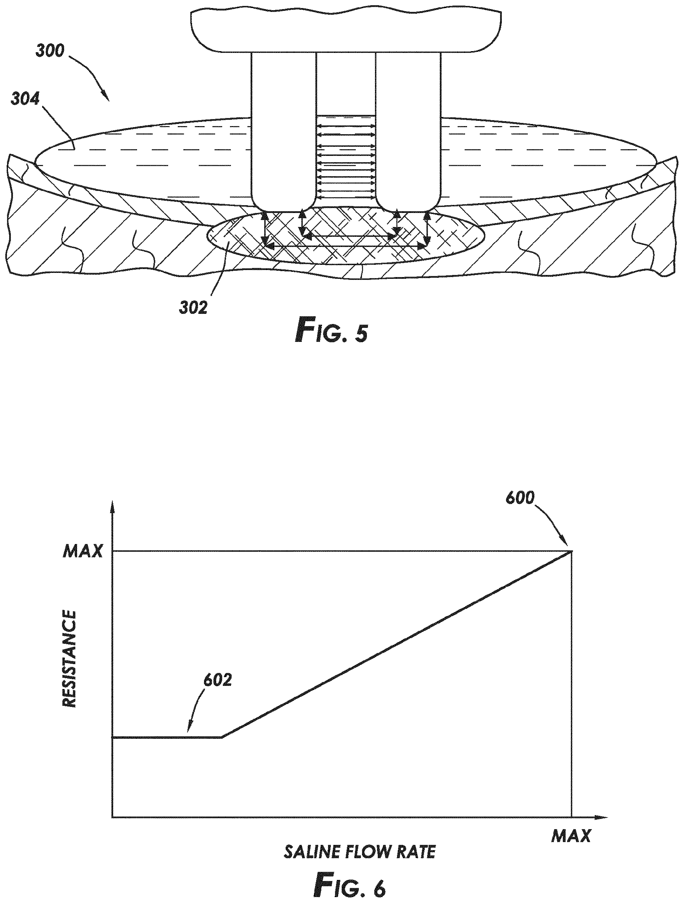

Figure 5 shows a simplified, partial cross-sectional side view, of a surgical site and a distal end of the coagulation wand. In particular, Figure 5 shows an example electrical current flow through the coagulation zone 302 as well as the saline 304 when the amount or depth of saline is excessive. Again, the electrodes 200 and 202 abut the tissue of the coagulation zone 302 and are at least partially submerged in the saline 304. However,

Figure 5 shows a situation in which the amount or depth of saline around the electrodes 200 and 202 has an overall resistance to current flow less than the resistance to current flow through the tissue at the surgical site 300, and thus the electrical current flows predominantly through the saline 304. Stated otherwise, Figure 4 shows a situation in which the amount or depth of saline around the electrodes 200 and 202 has an electrical conductivity greater than the electrical conductivity through the tissue at the surgical site 300, and thus the electrical current is shunted through the saline 304.

Having excess saline 304 at the surgical site may have several detrimental effects. For example, excess saline 304 may reduce the performance of the coagulation wand 104 because of lower electrical current flow through the tissue at the coagulation zone 302. The saline 304, acting as a shunt path for the electrical current, is heated by the current flow, and the saline quickly reaches temperatures that may burn or scald tissue within the surgical site 300 outside the coagulation zone 302. Further still, excess saline 304 may result in run-off of heated saline from the surgical site 300, which may cause damage to healthy tissue 306 (Figure 3) outside the surgical site 300. Moreover, excess saline 304 at the surgical site 300 may reduce visibility at the surgical site 300.

Various examples are directed to methods and systems of control of conductive fluid during coagulation, which control may address at least some of the possible detrimental effects noted above. In particular, in example systems the electrosurgical controller 116 applies RF energy between the electrodes 200 and 202 of the coagulation wand 104, the RF energy at the coagulation energy, and contemporaneously supplies a flow saline at an initial flow rate. During the application of RF energy, the electrosurgical controller 116 measures a value indicative of impedance of tissue and conductive fluid between the electrodes 200 and 202. The electrosurgical controller 116 is designed and constructed to decrease the flow of saline below the initial flow rate as the value indicative of impedance decreases. That is, as the amount or level of saline at the surgical site 300 increases, the impedance of the combined saline and tissue decreases. Thus, a decreasing impedance at the surgical site is indicative of increasing saline at the surgical site. Increased saline at the surgical site results in greater electrical current shunting through the saline, higher temperature saline, and an increased likelihood of saline run-off.

Oppositely, the electrosurgical controller 116 may be designed and constructed to increase the flow of saline as the value indicative of impedance increases. That is, as the amount or level of saline at the surgical site 300 decreases, the impedance of the combined saline and tissue increases. Thus, an increasing impedance at the surgical site is indicative of decreasing saline at the surgical site. Stated otherwise, as the conductivity at the surgical site decreases, the electrosurgical controller 116 is designed and constructed to increase the flow of saline to the surgical site.

Referring again to Figures 1 and 2. In many cases, a coagulation procedure is relatively short procedure (e.g., 30 seconds or less, in many case 15 seconds or less). For example, a clinician may cut tissue using a scalpel or electrosurgical cutter, and then reduce the bleeding from the newly exposed tissue by "painting" the tissue with the active coagulation wand 104. Consider, as a first example, a coagulation procedure on tissue that forms a crucible or bowl shape with respect to the direction of gravity. When the clinician triggers the coagulation procedure (e.g., using foot switch 138/140, or pressing button 150), the electrosurgical controller 116 supplies saline to the distal end 108 of the coagulation wand 104 and supplies RF energy to the electrodes 200 and 202. Because of the bowl shape in the example procedure, the saline tends to pool. The electrosurgical controller 116 measures the value indicative of the impedance, and because of the pooling the value indicative of impedance decreases over time. The electrosurgical controller 116 thus decreases the flow of saline from an initial flow rate as the value indicative impedance decreases - in this first example only a decrease in the flow of saline is implemented.

Now consider, as a second example, a coagulation procedure on tissue that forms a steep sidewall having a steep slope with respect to the direction of gravity. When the clinician triggers the coagulation procedure, the electrosurgical controller 116 supplies saline to the distal end 108 of the coagulation wand 104 and supplies RF energy to the electrodes 200 and 202. Because of the steep slope in the example procedure, the saline flows away from the coagulation zone under the force of gravity. The electrosurgical controller 116 measures the value indicative of the impedance; however, because the saline flows away from the coagulation zone, a steady-state wetting of the electrodes occurs, and thus the value indicative of impedance is relatively constant. The electrosurgical controller 116 may thus make no change in the flow of saline from the initial flow rate.

Now consider, as a third example, a coagulation procedure that begins with tissue forming a bowl shape, and then continues to contiguous tissue that forms a steep sidewall. When the clinician triggers the coagulation, the electrosurgical controller 116 supplies saline to the distal end 108 of the coagulation wand 104 and supplies RF energy to the electrodes 200 and 202. Because the coagulation starts within the bowl shape, initially the saline tends to pool. The electrosurgical controller 116 measures the value indicative of the impedance, and because of the pooling the value indicative of impedance decreases over time. The electrosurgical controller 116 thus decreases the flow of saline from an initial flow rate as the value indicative impedance decreases. However, as the coagulation procedure moves to the steep sidewall, the electrodes 200 and 202 depart the pooled saline, and the saline supplied to the distal end 108 of the coagulation wand 104 tends to flow away from the coagulation zone under the force of gravity. Thus, as the coagulation transitions from the bowl shape to the steep sidewall, the value indicative of the impedance increases. The electrosurgical controller 116 may thus increase flow the saline relative to the flow used when the distal end 108 of the coagulation wand 104 was within the bowl shape.

Now consider, as a fourth example, a coagulation procedure that begins on tissue that forms a steep side wall, and then continues to tissue forming a bowl shape. When the clinician triggers the coagulation, the electrosurgical controller 116 supplies saline to the distal end 108 of the coagulation wand 104 and supplies RF energy to the electrodes 200 and 202. Because of the coagulation starts with the steep side wall, there is no pooling around the electrodes 200 and 202. The electrosurgical controller 116 measures the value indicative of the impedance; however, because the saline initially flows away from the coagulation zone, a steady-state wetting of the electrodes occurs, and thus the value indicative of impedance is relatively constant. The electrosurgical controller 116 thus initially makes no change to the flow of saline from the initial flow rate. However, as the coagulation moves to the bowl shape, the electrodes 200 and 202 may be moved into pooled saline, and/or the saline begins to pool. Thus, as the coagulation transitions from the steep sidewall to the bowl shape, the value indicative of the impedance decreases. The electrosurgical controller 116 may thus decrease the flow the saline relative to the flow used when the distal end 108 of the coagulation wand 104 was associated with the steep sidewall.

It follows from the examples above that the control of the flow of saline by the electrosurgical controller 116 is not a closed-loop response attempting to achieve a setpoint impedance or a setpoint flow rate. Rather, the control of the flow of saline by the electrosurgical controller 116 may only be functional or active in situations in which the saline pools around the electrodes 200 and 202, and/or the coagulation transitions from an area where saline is pooling to a non-pooling area. Stated otherwise, the control of the flow of saline is operable to increase performance of the coagulation in the case of pooling and to reduce the likelihood of runoff of heated saline.

The value indicative of impedance measured by the electrosurgical controller 116 may take many forms. In one example, the value indicative of impedance may be the impedance of the parallel current paths through the saline 304 and tissue at the coagulation zone 302. In such cases, the electrosurgical controller 116 may be designed and constructed to increase the flow of saline as the magnitude of the impedance increases, and decrease the flow of saline as the magnitude of the impedance decreases.

As another example, the value indicative of impedance may be the admittance (e.g., the reciprocal of the impedance) of the combined current paths through the saline 304 and tissue at the coagulation zone 302. In such cases, the electrosurgical controller 116 may be designed and constructed to increase the flow of saline as the magnitude of the admittance decreases, and decrease the flow of saline as the magnitude of the admittance increases.

In other cases, the value indicative of impedance may be the resistance (e.g., the real part of the impedance) of the parallel current paths through the saline 304 and tissue at the coagulation zone 302. In such cases, the electrosurgical controller 116 may be designed and constructed to increase the flow of saline as the resistance increases, and decrease the flow of saline as the resistivity decreases.

In yet still other cases, the value indicative of impedance may be the conductance (e.g., the reciprocal of the resistance) of the combined current paths through the saline 304 and tissue at the coagulation zone 302. In such cases, the electrosurgical controller 116 may be designed and constructed to increase the flow of saline as the conductance decreases, and decrease the flow of saline as the conductance increases.

In some examples, the electrosurgical controller 116 is a constant voltage surgical controller, meaning that the electrosurgical controller 116 delivers RF energy to the electrodes of the coagulation wand 104 at a constant peak voltage when the voltage is applied. For example, a predetermined voltage is turned on and off at a radio frequency, and the duty cycle may be fixed or a variable. In such cases, the applied voltage is known, and thus electrical current through the combined current paths through the saline 304 and tissue at the coagulation zone 302 is proportional to the impedance presented. It follows that, in yet still other cases implemented using a constant voltage surgical controller, the value indicative of impedance may be a measurement of electrical current provided to the surgical site. In such cases, the electrosurgical controller 116 may be designed and constructed to increase the flow of saline as the electrical current decreases, and decrease the flow of saline as the electrical current increases. With respect to the electrosurgical controller 116 acting as a constant voltage surgical controller, the aggressiveness of the coagulation may be controlled by the applied voltage. That is, even though the applied voltage may be selected to implement an aggressiveness of coagulation, once set the applied peak voltage remains constant, and such shall not obviate the status of an electrosurgical controller 116 as a "constant voltage surgical controller." The specification now turns to example relationships between changes in the value indicative of impedance, and the changes to the flow of conductive fluid.

Figure 6 shows a plot of an example relationship of resistance and saline flow rate. In particular, the ordinate or "Y" axis of Figure 6 shows a value indicative of impedance in the example form of resistance, and the abscissa or "X" axis of Figure 6 shows saline flow rate. For the example electrosurgical controller 116 that implements a pump system in the form of a peristaltic pump or a positive displacement pump, saline flow rate may be equivalently stated as pump speed. In example systems, the saline flow rate may have an upper limit, designated as "max" in the plot as shown as point 600. That is the say, when the example resistance is at a highest value, the flow of saline may be set at the upper flow rate. As the example resistance drops, so too does the saline flow rate as shown in the plot of Figure 6. The example electrosurgical controller 116 may cease the flow of saline once the example resistance falls below a predetermined threshold, as shown by line 602. That is to say, once the example resistance falls below the predetermined threshold, the electrosurgical controller 116 may command the pump to cease or stop the delivery of saline. The saline flow rate may also track the plot of Figure 6 as the example resistance rises from a low value to higher values, including transitioning from zero saline flow rate to a non-zero saline flow rate as the example resistance transitions through the predetermined threshold.

The relationship between the value indicative of impedance in the example form of resistance and the saline flow rate of Figure 6 is, above the predetermined threshold, a directly proportional relationship, and in particular a straight line relationship. Moreover, the relationship is linear in the sense that there are no discontinuities in the relationship. Other relationships may be implemented.

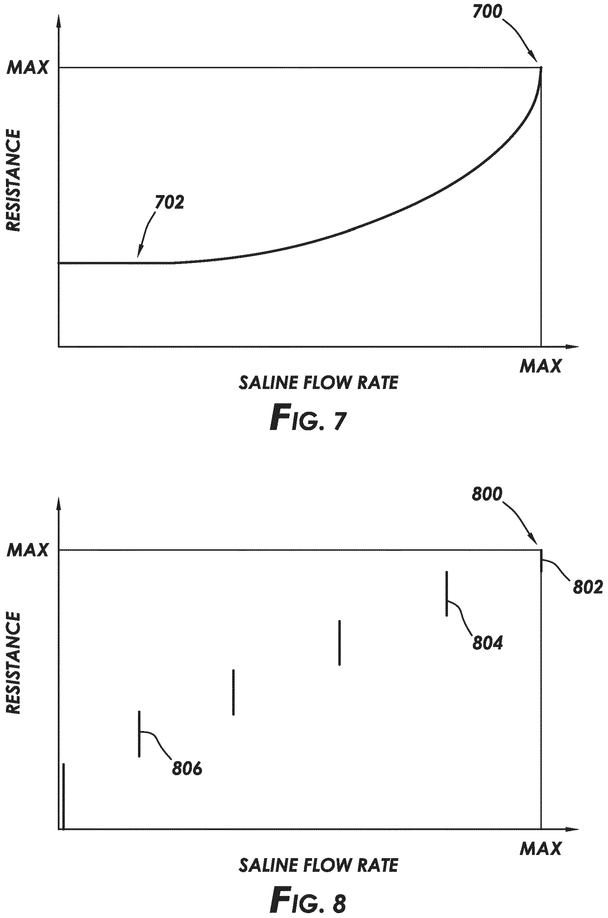

Figure 7 shows a plot of an example relationship of resistance and saline flow rate. In particular, the ordinate or "Y" axis of Figure 7 shows a value indicative of impedance in the example form of resistance, and the abscissa or "X" axis of Figure 7 shows saline flow rate. As before, for an electrosurgical controller 116 that implements a pump system in the form of a peristaltic pump or a positive displacement pump, saline flow rate may be equivalently stated as pump speed. As before, the saline flow rate may have an upper limit, designated as "max" in the plot as shown as point 700. That is the say, when the example resistance is at a highest value, the flow of saline may be set at the upper flow rate. As the example resistance drops, so too does the saline flow rate as shown in the plot of Figure 7. The example electrosurgical controller 116 may cease the flow of saline once the example resistance falls below the predetermined threshold, as shown by line 702. That is to say, once the example resistance falls below the predetermined threshold, the electrosurgical controller 116 may command the pump to cease or stop delivery of saline. The saline flow rate may also track the plot of Figure 7 as the example resistance rises from a low value to higher values, including transitioning from zero saline flow rate to a non-zero saline flow rate as the example resistance transitions through the predetermined threshold.

The relationship between the value indicative of impedance in the example form of resistance and the saline flow rate of Figure 7 is, above the predetermined threshold, a directly proportional relationship, and in particular an exponential relationship. Moreover, the relationship is linear in the sense that there are no discontinuities in the relationship. However, yet still other relationships may be implemented.

Figure 8 shows a plot of an example relationship of resistance and saline flow rate. In particular, the ordinate or "Y" axis of Figure 8 shows a value indicative of impedance in the example form of resistance, and the abscissa or "X" axis of Figure 8 shows saline flow rate. As before, the example electrosurgical controller 116 that implements a pump system in the form of a peristaltic pump or a positive displacement pump, saline flow rate may be equivalently stated as pump speed. As before, the saline flow rate may have an upper limit, designated as "max" in the plot as shown as point 800. As the example resistance drops, so too does the saline flow rate; however, in the example of Figure 8 defines a plurality of example resistance ranges for which the saline flow rate is constant. For example, if the example resistance is within a first range 802, the saline flow rate may be constant for all resistances within the first range 802 as illustrated by Figure 8. If the example resistance is within a second range 804, the saline flow rate may be constant for all resistances within the second range 804. The step-wise relationship continues for decreasing values of the example resistance. If the example resistance is within a fifth range 806, the saline flow rate may be constant for all resistances within the fifth range 806. Finally, the example electrosurgical controller 116 may cease the flow of saline once the example resistance falls below the predetermined threshold (e.g., the lowest resistance value of the fifth range 806). That is to say, once the example resistance falls below the predetermined threshold, the electrosurgical controller 116 may command the pump to cease or stop delivery of saline. The saline flow rate may also track the step-wise plot of Figure 8 as the example resistance rises from a low value to higher values, including transitioning from zero saline flow rate to a non-zero saline flow rate as the example resistance transitions through the predetermined threshold.

The relationship between the value indicative of impedance in the example form of resistance and the saline flow rate of Figure 8 is, above the predetermined threshold, a directly proportional relationship in a step-wise sense. Moreover, the relationship may be considered piece-wise linear. One having ordinary skill, now understanding the example relationships of the value indicative of impedance and the saline flow rate, understands the various examples may be combined in many forms (e.g., the exponential relationship for higher values of resistance, straight line for mid-range values of resistance, and step-wise for lower-range values of the resistance).

In yet still further examples, the electrosurgical controller 116 may be designed and constructed to only decrease the flow of conductive fluid. That is, in some cases each coagulation may begin at an initial flow. The initial flow rate may be the upper limit flow rate shown in Figures 6-8, but in other cases may be below the upper limit flow rate. Nevertheless, in example systems if the value indicative of impedance decreases, the flow of saline may decrease according to any of the relationships shown and/or discussed above, including in some cases ceasing the flow of saline. However, even if the value indicative of impedance again rises (e.g., the clinician transitions the distal end 108 of the coagulation wand 104 out of the pool of saline), no increase in the flow of saline is implemented. To the extent the clinician desires further saline flow, the coagulation may be stopped by releasing the foot pedal 138/140 or button 150, thus ending the application of RF energy, and then started anew by once again pressing the foot pedal 138/140 or button 150.

The various examples discussed to this point implicitly assume that the RF energy remains constant during coagulation, or for constant voltage surgical controllers changes only responsive to changes in impedance presented by the saline and tissue of the coagulation zone. However, in yet still further cases the example electrosurgical controller 116 may be designed and constructed to decrease the RF energy applied to the electrodes 200 and 202 as the value indicative of impedance decreases. Consider, as an example, the situation from above in which a coagulation takes place on tissue that forms a crucible or bowl shape with respect to the direction of gravity. Because of the bowl shape, the saline tends to pool in the bowl shape. As the depth of the saline increases, the value indicative of impedance decreases. The example electrosurgical controller 116 decreases the flow of saline in any of the example forms discussed above, including decreasing to zero. If the value indicative of impedance continues to decrease (e.g., as the saline temperature continues to rise), the example electrosurgical controller 116 may be further designed and constructed decrease the RF energy supplied, including in some cases ceasing the application of RF energy. Decreasing the RF energy supplied may take any suitable form. For example, the electrosurgical controller 116 may decrease applied voltage. In other cases, the electrosurgical controller 116 may decrease the on-time of each application of energy (e.g., decrease the duty cycle of the applied energy). In yet still other cases, the electrosurgical controller 116 may both reduce the applied voltage and reduce the duty cycle. Regardless of the precise method, the RF energy may be decreased.

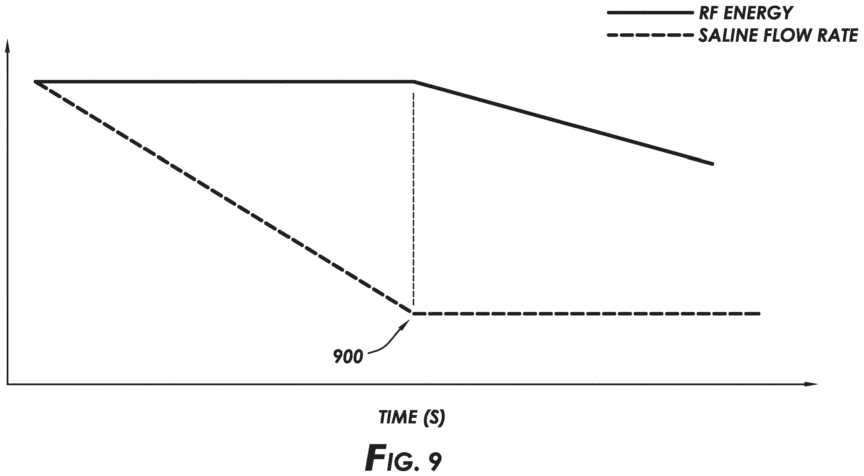

Figure 9 shows an example plot of saline flow rate and applied RF energy over time. In particular, Figure 9 shows a situation in which the saline flow rate is reduced over time to a lowest flow rate at time 900. The lowest flow rate is shown in Figure 9 as non-zero, but in other cases the lowest flow rate may be zero. In the example of Figure 9, the RF energy delivered is constant during the example duration in which the flow rate is decreasing. In the example of Figure 9, when the saline flow rate is the lowest flow rate at time 900, the example electrosurgical controller 116 may be designed and constructed to lower the RF energy applied over time to a lowest RF energy, including in some cases a zero RF energy (e.g., the coagulation ends). Figure 9 shows the RF energy declining over time in a straight line sense, but any suitable reduction rate may be used (e.g., exponential decay, piece-wise linear decay). Moreover, while Figure 9 shows the reduction in RF energy occurring after the saline flow rate reaches its lowest point, the electrosurgical controller 116 may be designed and constructed to begin the reduction in RF energy at any suitable point in time (e.g., when the value indicative of impedance falls through an boundary threshold), including reducing the RF energy before the saline flow rate reaches its lowest value. The specification now turns to an example internal structure of the electrosurgical controller 116.

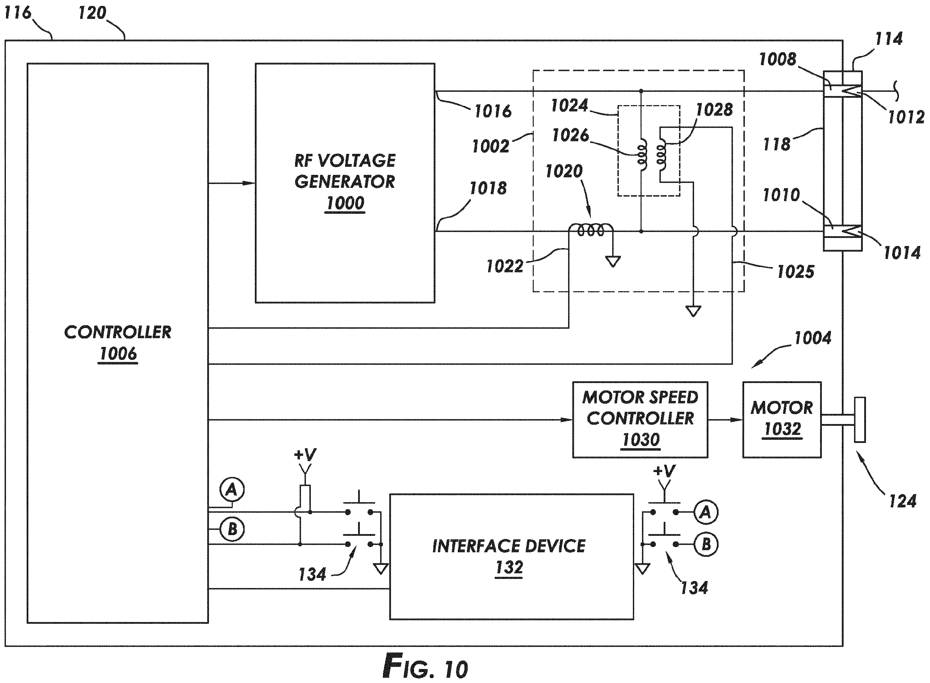

Figure 10 shows a block diagram of an example electrosurgical controller 116. The example electrosurgical controller 116 comprises the enclosure 120, the controller connector 118, an RF voltage generator 1000, a measurement circuit 1002, a pump system 1004, and a controller 1006. The example controller connector 118 is accessible on an external surface of the enclosure 120. Disposed within the controller connector 118 are electrical pins 1008 and 1010. The wand connector 114 is also shown in Figure 10, including corresponding electrical pins 1012 and 1014 in the wand connector 114. Thus, when the wand connector 114 is mechanically coupled to the controller connector 118, the electrical pins 1012 and 1014 electrically couple to the electrical pins 1008 and 1010, respectively.

The example RF voltage generator 1000 defines an active lead or active terminal 1016 which is coupled to the electrical pin 1008 in the controller connector 118, and defines a return lead or return terminal 1018 which is coupled to the electrical pin 1010 in the controller connector 118. When the wand connector 114 is coupled to the controller connector 118, the active terminal 1016 is coupled electrode 200 (Figure 2) and the return terminal is coupled to the electrode 202 (or vice versa). Additional active terminals and/or return terminals may be used. The active terminal 1016 may be the terminal upon which the voltages and electrical currents are induced by the RF voltage generator 1000 relative to the return terminal 1018, and the return terminal 1018 provides a return path for electrical currents. In many cases, and for patient safety, the RF voltage generator 1000 is electrically floated, and the RF energy provided is an AC signal, and thus the designation as active and return may be reversed.

The RF voltage generator 1000 is configured to produce coagulation energy at a coagulation frequency. In accordance with example embodiments, the coagulation frequency produced by the RF voltage generator 1000 may be between about 5 kHz and 20 MHz, in some cases between about 30 kHz and 2.5 MHz, in other cases between about 50 kHz and 500 kHz, and in a particular case about 100 kHz. The coagulation voltage may be between and including 120V and 165V RMS. In the example system shown the RF voltage generator 1000 is communicatively coupled to the controller 1006, and the RF voltage generator 1000 receives commands from the controller 1006 in any suitable form, such as an analog signal (e.g., produced by a digital-to-analog converter, not specifically shown), digitally (e.g., by way of digital outputs, not specifically shown), or by way of packet-based messages (e.g., by way of a communication logic, not specifically shown).

Disposed electrically between the RF voltage generator 1000 and the controller connector 118 is the measurement circuit 1002. The example measurement circuit 1002 defines a current sensing device, illustratively shown as current transformer 1020 defining sense lead 1022. The "primary" of the current transformer 1020 is the return terminal 1018, but in other cases the "primary" may be the active terminal 1016. The sense lead 1022 of the example current transformer 1020 is coupled to the controller 1006. Thus, the controller 1006 reads a signal indicative of current flow, produces a value indicative of current flow, and further may produce the value indicative of impedance (which in some cases may be the value indicative of current flow). In cases in which the controller 1006 is a processor, microcontroller, PSOC, or the like, the controller may implement an analog-to-digital converter to read the signal indicative of current flow; however, in other cases the analog-to-digital converter may be a stand-alone device collectively a member of the controller 1006. Other current sensing devices may be used to create the signal indicative of current flow, such as Hall-effect current sensors, or low value sense resistors placed in series with the electrical current flow.

The example measurement circuit 1002 further defines an isolation transformer 1024 to sense the voltage applied across the electrical pins 1008 and 1010. The example isolation transformer 1024 defines a primary winding 1026 coupled across the active terminal 1016 and the return terminal 1018. The example isolation transformer 1024 further defines a secondary winding 1028 magnetically coupled to the primary winding 1026. The sense lead 1025 of the secondary winding 1028 is electrically coupled to the controller 1006. In operation, the controller 1006 senses voltage applied to the across the electrical pins 1008 and 1010 (and thus the electrodes 200 and 202). That is, the controller 1006 reads a signal indicative of applied voltage, produces a value indicative of applied voltage, and may further produce the value indicative of impedance based on the applied voltage. In cases where the controller 1006 is a processor, microcontroller, or PSOC, or the like, the controller 1006 may implement an analog-to-digital converter to read the signal indicative of applied voltage; however, in other cases the analog-to-digital converter may be a stand-alone device collectively a member of the controller 1006.

Turning to the pump system 1004. The example pump system 1004 comprises the peristaltic pump 124. In order to control rotational speed of the peristaltic pump 124, the example electrosurgical controller 116 implements a motor speed controller 1030 communicatively coupled to the controller 1006, and operationally coupled to a motor 1032, where the peristaltic pump 124 is turned by the motor 1032. The motor 1032 may take any suitable form. For example, the motor 1032 may be a DC electric motor, and thus the motor speed controller 1030 provides a DC voltage to the electric motor which controls the rotational speed of the output shaft. In other cases, the motor 1032 may be an AC electric motor, and thus the motor speed controller 1030 provides at varying AC voltage and frequency which controls the rotational speed of the output shaft. In yet still other cases, the motor 1032 may be a pneumatic motor, and thus the motor speed controller 1030 provides air at varying pressures, where the pressure controls the rotational speed of the output shaft. Thus, regardless of the type of motor 1032 implemented, the motor speed controller 1030 controls the rotational speed of the output shaft of the motor 1032 responsive to commands provided from the controller 1006. The controller 1006 may communicatively couple to the motor speed controller 1030 in any suitable form, such as an analog signal (e.g., produced by a digital-to-analog converter, not specifically shown), digitally (e.g., by way of digital outputs, not specifically shown), or by way of packet-based messages (e.g., by way of a communication logic, not specifically shown). Finally, while the motor 1032 is shown to directly couple to the peristaltic pump 124, in other cases various gears and/or belts may be used to transfer the rotational motion of the shaft of the motor 1032 to peristaltic pump 124. While Figure 21 is based on having a rotary peristaltic pump, one having ordinary skill, and with the benefit of this disclosure, could modify the system to be used with other types of pumps, such as linear peristaltic pumps, a positive displacement pumps, or a centrifugal pumps combined with flow a measurement device (as the flow rate through a centrifugal pumps is not as directly related to speed).

It is noted that Figure 10 shows the peristaltic pump 124 as an internal or integral device within the enclosure 120 of the electrosurgical controller 116; however, in other cases the peristaltic pump 124 may be an external component to the electrosurgical controller 116, yet still operationally connected to the electrosurgical controller 116. Moreover, while only one pump system 1004 is shown in Figure 10, a coagulation controller may implement two or more pump systems (e.g., a second pump to control aspiration).

Still referring to Figure 10, the example electrosurgical controller 116 further includes the interface device 132 communicatively coupled to the controller 1006. The interface device 132 may be a display device in any suitable form (e.g., LCD screen). Figure 10 also shows example buttons 134 associated with the interface device 132. The example buttons 134 each couple to the controller 1006, such as by way of digital inputs implemented by the controller 1006.

The example controller 1006 may take any suitable form. For example, the controller may comprise individual circuit components (e.g., an analog or digital circuit created from individual components). The example controller 1006 may be an application specific integrated circuit (ASIC). The example controller 1006 may be programmable device, such as a processor, a microcontroller, a reduced-instruction-set computing (RISC) device, a digital signal processor (DSP), a programmable logic device (PLD), a field programmable gate array (FPGA), or a programmable system-on-a-chip (PSOC). In yet still other cases, the controller 1006 may be combinations of any of the recited components.

The example electrosurgical controller 116 of Figure 10 may have additional components and features that are not shown so as not to unduly complicate the figure. For example, the internal and external components associated with the pedal connector 144 are not shown. Various other power supply modules, on/off switches, and overcurrent protection devices would also be present, but again such devices are not shown so as not to unduly complicate the figure.

The controller 1006 may thus be designed and constructed to communicatively interface with the various other components to implement the functionality described above. For example, in the context of a coagulation, the controller 1006 may be designed and constructed to command the RF voltage generator 1000 to supply RF energy across the electrical pins 1008 and 1010 of the controller connector 118, and simultaneously command the pump system 1004 to supply a flow of conductive fluid. During periods of time in which RF energy is being provided and there is a flow of conductive fluid, the controller 1006 may read a signal indicative of impedance from the measurement circuit 1002, and create a value indicative of impedance in any of the example forms discussed above. Based on the value indicative of impedance, the controller 1006 may command the pump system 1004 to decrease the flow of conductive fluid as the value indicative of impedance decreases. Oppositely, in some cases, the controller 1006 may also be designed and constructed to command the pump system to increase the flow of conductive fluid as the value indicative of impedance increases. Further still, the example controller 1006 may be designed and constructed to command the pump system to cease the flow of conductive fluid when the value indicative of impedance falls below a predetermined threshold, and possibly to command the pump system to resume the flow of conductive fluid when the value indicative of impedance rises above the predetermined threshold.

Further still, particularly in cases in which the electrosurgical controller 116 is used for procedures associated with the nasopharynx, the controller 1006 may be designed and constructed to generate an alarm when the value indicative of impedance remains below the predetermined threshold for a predetermined duration. The value indicative of impedance falling below the predetermined threshold for a predetermined duration may be indicative of a clog in the aspiration tubing associated with the procedure. The aspiration tubing may be a standalone aspiration device, or the aspiration tubing may be integral with the coagulation device.

Yet further still, in cases in which the controller 1006 controls not only on-off status of the RF voltage generator 1000, but can also control the RF energy provided by the RF voltage generator 1000 (e.g., the applied or peak voltage generated), the example controller 1006 may be designed and constructed to command the RF voltage generator to decrease the RF energy as the value indicative of impedance decreases. Oppositely, in some cases, the controller 1006 may also be designed and constructed to command the RF voltage generator to increase the RF energy as the value indicative of impedance increases.

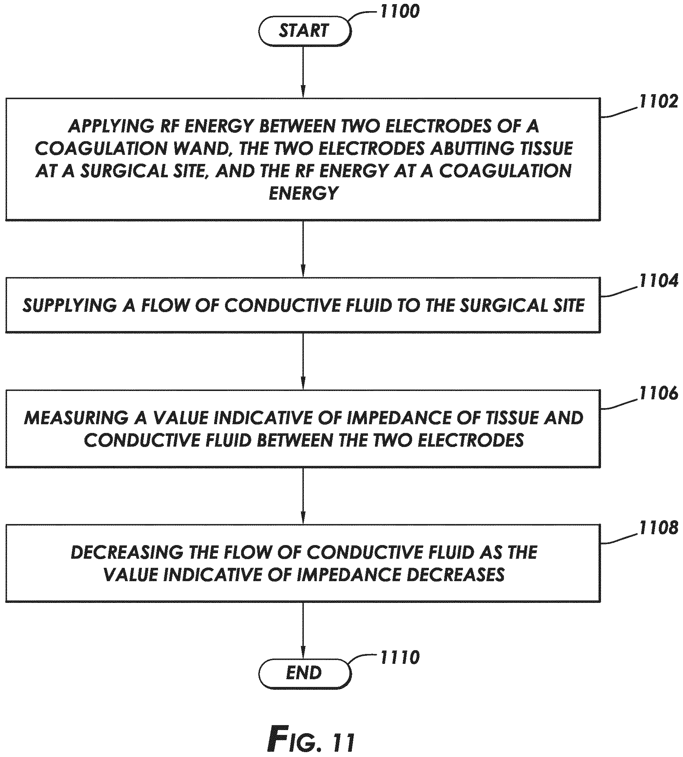

Figure 11 shows a method in accordance with at least some embodiments. The example method may be implemented, in whole or in part, by software executing in the controller 1006 of the electrosurgical controller 116. In particular, the method starts (block 1100) and comprises: applying radio frequency (RF) energy between two electrodes of a coagulation wand, the two electrodes abutting tissue at a surgical site, and the RF energy at a coagulation energy (block 1102); supplying a flow of conductive fluid to the surgical site (block 1104); measuring a value indicative of impedance of tissue and conductive fluid between the two electrodes (block 1106); and decreasing the flow of conductive fluid as the value indicative of impedance decreases (block 1108). Thereafter, the method ends (block 1110).

The above discussion is meant to be illustrative of the principles and various embodiments of the present invention. Numerous variations and modifications will become apparent to those skilled in the art once the above disclosure is fully appreciated. It is intended that the following claims be interpreted to embrace all such variations and modifications.

## Claims

1. A method of sealing blood vessels, the method comprising:
applying, by an electrosurgical controller, radio frequency (RF) energy between two electrodes of a coagulation wand, the two electrodes abutting tissue at a surgical site, and the RF energy at a coagulation energy;
supplying, by the electrosurgical controller, a flow of conductive fluid to the surgical site;
measuring, by the electrosurgical controller, a value indicative of impedance of tissue and conductive fluid between the two electrodes; and
decreasing, by the electrosurgical controller, the flow of conductive fluid as the value indicative of impedance decreases.

2. The method of claim 1 further comprising ceasing the flow of conductive fluid when the value indicative of impedance falls below a predetermined threshold.

3. The method of claim 2 further comprising generating an alarm, by the electrosurgical controller, when the value indicative of impedance remains below the predetermined threshold for a predetermined duration, the alarm indicating a clog in an aspiration tubing associated with the surgical site.

4. The method of claim 1 further comprising increasing, by the electrosurgical controller, the flow of conductive fluid as the value indicative of impedance increases.

5. The method of claim 1:
wherein measuring the value indicative of impedance further comprises measuring resistance between the two electrodes;
wherein decreasing the flow of conductive fluid further comprises decreasing the flow of conductive fluid as the resistance decreases.

6. The method of claim 1 wherein decreasing the flow of conductive fluid further comprising at least one selected from a group comprising: decreasing directly proportional to the value indicative of impedance; decreasing linearly proportional to the value indicative of impedance; decreasing step-wise proportional to the value indicative of impedance.

7. The method of claim 1 wherein applying the RF energy further comprises decreasing the RF energy as the value indicative of impedance decreases.

8. The method of claim 1 wherein measuring the value indicative of impedance further comprises measuring at least one selected from a group comprising: impedance; the real part of the impedance; resistance; electrical current flow for constant voltage surgical controller providing the RF energy.

9. An electrosurgical controller comprising:
a connector accessible on an external surface of a housing, the connector defines two electrical pins;
a radio frequency (RF) voltage generator disposed within the housing, and electrically coupled to the two electrical pins of the connector;
a measurement circuit disposed within the housing and electrically connected between the RF voltage generator and the two electrical pins of the connector, the measurement creates a signal indicative of impedance;
a pump system configured to pump conductive fluid to a surgical site; and
a controller disposed within the housing and communicatively coupled to the measurement circuit and the pump system, the controller configured to:
command the RF voltage generator to supply RF energy across the two electrical pins of the connector, and the RF energy at a coagulation energy;
command the pump system to supply a flow of conductive fluid;
read the signal indicative of impedance from the measurement circuit and create a value indicative of impedance; and
command the pump system to decrease the flow of conductive fluid as the value indicative of impedance decreases.

10. The electrosurgical controller of claim 9 wherein the controller is further configured to command the pump system to cease the flow of conductive fluid when the value indicative of impedance falls below a predetermined threshold.

11. The electrosurgical controller of claim 10 wherein the controller is further configured to generate an alarm when the value indicative of impedance remains below the predetermined threshold for a predetermined duration.

12. The electrosurgical controller of claim 9 wherein when the controller reads the signal indicative of impedance and creates the value indicative of impedance, the controller is configured to create at least one selected from a group comprising: a value indicative of resistance; a value indicative the real part of the impedance; and a value indicative of electrical current.

13. The electrosurgical controller of claim 9 wherein when the controller commands the pump system to decrease the flow of conductive fluid, the controller is further configured to command the decrease the flow of conductive fluid: directly proportional to the value indicative of impedance; linearly proportional to the value indicative of impedance; and step-wise proportional to the value indicative of impedance.

14. The electrosurgical controller of claim 9 wherein the controller is further configured to command the pump system to increase the flow of conductive fluid as the value indicative of impedance increases.

15. The electrosurgical controller of claim 9 wherein when the controller commands the RF voltage generator to supply RF energy, the controller is further configured to command the RF voltage generator to decrease the RF energy as the value indicative of impedance decreases.

16. An electrosurgical coagulation system comprising:
a source of conductive fluid;
a coagulation wand defining first and second electrodes disposed at a distal end of the coagulation wand and a delivery lumen associated with a distal end of the coagulation wand;
an electrosurgical controller comprising:
a radio frequency (RF) voltage generator electrically coupled to the first and second electrodes of the coagulation wand;
an impedance sensitive circuit electrically connected between the RF generator and the first and second electrodes of the coagulation wand, the impedance sensitive circuit creates a signal indicative of impedance;
a pump system fluidly coupled to the source of conductive fluid and the delivery lumen; and
a controller electrically coupled to the impedance measuring circuit and the pump system, the controller configured to:
command the RF voltage generator to supply RF energy between the first and second electrodes of the coagulation wand, and the RF energy at a coagulation energy;
command the pump system to supply a flow of conductive fluid;
read the signal indicative of impedance from the impedance sensitive circuit and create a value indicative of impedance; and
command the pump system to decrease the flow of conductive fluid to the delivery lumen as the value indicative of impedance decreases.

17. The electrosurgical coagulation system of claim 16 wherein the controller is further configured to command the pump system to cease the flow of conductive fluid to the delivery lumen when the value indicative of impedance falls below a predetermined threshold.

18. The electrosurgical coagulation system of claim 17 wherein the controller is further configured to generate an alarm when the value indicative of impedance remains below the predetermined threshold for a predetermined duration, the alarm indicating a clog in an aspiration tubing associated with a surgical site.

19. The electrosurgical coagulation system of claim 16 wherein when the controller reads the signal indicative of impedance and creates the value indicative of impedance, the controller is configured to create at least one selected from a group comprising: a value indicative of resistance; a value indicative the real part of the impedance; and a value indicative of electrical current.

20. The electrosurgical coagulation system of claim 16 wherein when the controller commands the pump system to decrease the flow of conductive fluid, the controller is further configured to command the decrease of the flow of conductive fluid: directly proportional to the value indicative of impedance; linearly proportional to the value indicative of impedance; and step-wise proportional to the value indicative of impedance.

21. The electrosurgical coagulation system of claim 16 wherein the controller is further configured to command the pump system to increase the flow of conductive fluid to the delivery lumen as the value indicative of impedance increases.

22. The electrosurgical coagulation system of claim 16 wherein when the controller commands the RF voltage generator to supply RF energy, the controller is further configured to command the RF voltage generator to decrease the RF energy as the value indicative of impedance decreases.
